# EUROPEAN PATENT SPECIFICATION

(11) **EP 2 957 280 B1**
(45) Date of publication and mention of the grant of the patent: **17.04.2019**
(21) Application number: 15001790.3
(22) Date of filing: 17.06.2015
(51) Int. Cl.: A61K 31/198, A61K 9/20

(54) **SOLID PHARMACEUTICAL COMPOSITION OF CYTISINE AND PROCESS FOR PREPARATION THEREOF**
PHARMAZEUTISCHE FESTSTOFFZUSAMMENSETZUNG VON CYTISIN UND VERFAHREN ZUR HERSTELLUNG DAVON
COMPOSITION PHARMACEUTIQUE SOLIDE DE CYTISINE ET SON PROCÉDÉ DE PRÉPARATION

(30) Priority: 18.06.2014 PL 40860814
(43) Date of publication of application: 23.12.2015
(73) Proprietor: Bonteque Consulting LTD, London W5 2BS (GB)
(72) Inventor: Berdzinska, Katarzyna, 62-095 Murowana Goslina (PL); Kieronska, Hanna, 62-051 Wiry (PL); Milewski, Marek, 60-193 Poznan (PL); Nawrocka, Malgorzata, 60-718 Poznan (PL); Klepczynska, Marta, 11-700 Mragowo (PL)
(74) Representative: Gizinska-Schohe, Malgorzata

(56) References cited:
- EP-A1- 1 586 320
- EP-A1- 2 233 134
- WO-A1-2014/076680

## Description

### Technical Field

The present invention relates to a stable solid pharmaceutical composition of cytisine and process for the preparation thereof.

Cytisine is a plant alkaloid, which pharmacological properties have been cited since the 1970s. The description of patent PL185934 mentioned, that cytisine had been used as painkiller "cytiton" (source: Mashkovsky, M. D., "Medicine remedies", M., 1977, v.1, p.123). The description of international application WO8302892A1 published in 1983 mentioned, that in Bulgaria it had been manufactured the preparation called "Tabex", which contained cytisine and similar preparation manufactured in Yugoslavia called "Kuz'-manovich tablets".

There are medicinal products containing 1.5 mg of cytisine in a form of film-coated tablets and hard capsules used in treatment of nicotine addiction. In the description of the EP1586320 application there is presented cytisine containing solid dosage form being film-coated tablet, wherein the core contains lactose, microcrystalline cellulose, talc, and magnesium stearate. Appropriate lactose to microcrystalline cellulose ratios are also presented as 1:2.0 to 1:2.5. Tablet prepared according to the cited composition had better stability and mechanical strength than previously manufactured tablets containing i.e. lactose, wheat starch and calcium bicarbonate. The application EP1586320 does not present the type of manufacturing technology used for the preparation of the cytisine tablets nor whether it is possible to obtain tablet containing 1.5 mg of cytisine using direct compression process and fulfil the requirements presented in European Pharmacopoeia.

Tablets presented in the example of the EP1586320 application had adequate uniformity of the active substance, but it was not revealed whether good uniformity was an effect of prepared composition or appropriate choice of particle size of the active substance and excipients or the appropriately chosen technology. All these parameters may have an impact on the uniformity of content of the active substance.

In the Polish application P.401676 there is presented a composition in a form of hard capsule containing micronized cytisine in amount of 0.1% to 5%, wherein all the particles have diameter smaller than 10 µm. The composition contains also from 40% to 60% of corn starch and from 40% to 60% of microcrystalline cellulose.

### Background of the invention

Medicinal products have to comply with many quality requirements, that guarantee safety and efficacy of its use in the whole shelf-life. In case of solid dosage forms for oral administration of medicinal products important quality criteria comprise assay of the active substance, uniformity of content of the active substance and content of impurities. In case of tablets appropriate mechanical strength is also required.

Achieving uniform content of the active substance in the tablets manufactured using direct compression method is problematic when the active substance is present in only a few percent of the total tablet mass. In the process of blending of the dry blend segregation or aggregation of the active substance may take place. Moreover, dry blend must have good technological parameters (e.g. flowability), what allows to maintain constant conditions in the tabletting or encapsulation process.

Achieving better homogeneity of the tabletting blend is possible with the use of wet granulation process, which is however more complicated than direct compression process. Additionally, wet granulation in case of hygroscopic substances of good solubility in water, such as cytisine, may be related to technological difficulties, which arise from increase in viscosity of the granules and difficulties in water removal.

In turn, granulation with the use of organic solvent is less preferable from environmental reasons, needs special equipment for production line and enforces the necessity of testing residual solvents in the final product.

During development of the medicinal product containing cytisine it turned out that commercially available preparation in a form of film-coated tablets is not stable in the conditions of increased temperature and humidity. Tablets after taking out of blisters were tested under 30°C/65% RH; 40°C/75% RH, where RH represents relative humidity. After 28 days significant decrease of content of the active substance was observed.

The results of the content of the active substance in the product "Tabex" after 28 days of storage at specified conditions were summarized in the table 1.

**Table 1**

| Conditions | Percent of declared content [%] | Change relative to declared content [%] |
|---|---|---|
| 30°C/65% RH | 84.7% | -12.3% |
| 40°C/75% RH | 67.4% | -30.1% |

On the basis of the performed tests it was concluded that cytisine contained in the tested product undergoes significant degradations in the environment of increased humidity.

Additionally compatibility studies between active substance and different excipients were performed. Summary of the total impurities content observed in the mixture of micronized cytisine with specified excipients was presented in the table 2
The mixtures were stored at 50°C/75% RH for 28 days.

**Table 1**

| Composition of the mixture | Impurity content |
|---|---|
| Cytisine + lactose | 77.1% |
| Cytisine + silicified microcrystalline cellulose | 2.3% |
| Cytisine + microcrystalline cellulose | 1.7% |
| Cytisine + calcium hydrogen phosphate | 1.5% |
| Cytisine + sodium stearyl fumarate | 0.8% |
| Cytisine + magnesium stearate | 0.5% |
| Cytisine + colloidal silicon dioxide | 0.5% |
| Cytisine + croscarmellose sodium | 0.3% |
| Cytisine + mannitol | 0.2% |
| Cytisine + talc | 0.2% |
| Cytisine + starch | 0.1% |
| Cytisine (without any excipients) | 0.0% |

Compatibility studies revealed that lactose (Lactopress 250SD) is highly incompatible with cytisine, what puts into question a possibility of using lactose as excipient in the tables containing cytisine.

Smaller incompatibility was observed with silicified microcrystalline cellulose (Prosolv SMCC 50) and even smaller with microcrystalline cellulose (MCC PH101), calcium hydrogen phosphate (Emcompress) and sodium stearyl fumarate (Pruv). Unexpectedly it turned out that excipients such as: starch (Starch 1500), talc, mannitol (Parteck M100), sodium croscarmellose (Vivasol), colloidal silicon dioxide (Aerosil) and magnesium stearate were the most compatible with cytisine and did not contribute to significant degradation of cytisine in high humidity conditions.

Technical problem of preparation solid stable pharmaceutical compositions containing cytisine with the possibility to use direct compression technology was the basis of the present invention.

In particular, the object is achieved by a composition according to claim 1, and a process of claim 10. Preferred embodiments are subject of dependent claims.

### Summary of the invention

The subject of the present invention is a stable solid pharmaceutical composition of cytisine in a form of tablet manufactured by direct compression. Compositions manufactured according to the present invention are stable during long term storage, also under increased humidity conditions.

For the manufacturing of the composition, the above effects can be achieved by using active substance cytisine having adequately chosen particle size and from 20% to 75% by weight of microcrystalline cellulose, glidant and at least one excipient chosen from the group consisting of: mannitol, colloidal silicon dioxide, calcium hydrogen phosphate.

Compositions prepared according to the present invention do not contain lactose, whereby such compositions may be used in patients with lactose intolerance without additional risk of symptoms of gastrointestinal disorders.

High uniformity of cytisine content in tablets is preferably achieved by using cytisine, which at least 60% of particles have size from 10 µm to 200 µm. Preferably average particle size of cytisine d₅₀ is in the range from 10 µm to 30 µm, wherein particularly preferable is at least 90% of cytisine particles having size below 80 µm (value d₉₀ below 80 µm), Term "average particle size" refers to the volume mean diameter of particles. Volume mean diameter of particles was calculated by laser light diffraction method using Malvern Mastersizer 2000 apparatus. Value d₅₀ of 20 µm means that 50% of the particles by volume have diameter below 20 µm. Particularly high uniformity of the composition was achieved when diluent having average particle size d₅₀ within a range of 100 µm do 150 µm. Particularly preferable is when diluent having average particle size of 100 µm is present in amount of at least 20% of the mass of composition.

Unexpectedly it also turned out that even low water content in the active substance (below 2%) contributes significantly to reducing the stability of cytisine tablets manufacture using direct compression method. Cytisine used in the present invention contains preferably below 1% of water. The most preferable water content in the active substance is below 0.5%.

Water content was determined with the methods described in European Pharmacopoeia 8.0 in chapter 2.2.32 "Loss on drying" or with the Karl-Fischer method described in chapter 2.5.12 "Water: Semi-Micro Determination". Water content in the tablets prepared according to the present invention did not exceed 1%.

Compositions obtained according to the present invention are of high uniformity of cytisine content and comply with the European Pharmacopoeia 8.0 requirements described in chapters 2.9.6 "Uniformity of content of single-dose preparations" and 2.9.40 "Uniformity of dosage units".

### Detailed description of the invention

Composition according to the present invention preferably comprises:
a) from 0.1% to 3% by weight of the active substance;
b) from 20% to 75% by weight of microcrystalline cellulose;
c) from 20% to 77.5% by weight of diluent chosen from a group comprising mannitol, colloidal silicon dioxide, calcium hydrogen phosphate;
d) from 0% to 10% by weight of binder;
e) from 0% to 5% by weight of disintegrant;
f) from 0% to 2% by weight of anti-adhesion agent;
g) from 0.1% to 2.5% of glidant.

Single tablet prepared according to the present invention contains preferably 1.5 mg of cytisine.

Preferably formulation according to the present invention comprises at least one diluent, which is suitable for direct compression. Preferable diluents are mannitol, silicified microcrystalline cellulose, calcium hydrogen phosphate, starch. Apart from the diluent used, composition comprises optionally a binder. Binders are chosen from the group comprising such substances as powder cellulose, cellulose derivatives such as carboxymethylcellulose, hydroxypropylmethylcellulose, hydroxypropylcellulose. Content of the binder is within a range from 0% to 10% by weight. Total content of the diluent and binder is preferably within a range from 90% to 98.5% by weight. It is preferable for the preparation of the composition according to the present invention to use a blend for which ratio of microcrystalline cellulose to mannitol is from 1:4 to 1:2.

Optionally composition according to the present invention comprise anti-adhesion agent. Anti-adhesion agent are preferable chosen from a group comprising colloidal silicon dioxide, hydrophilic fumed silica (Aerosil 200), talc. Content of the anti-adhesion agent is preferably within a range from 0% to 2%.

Preferably composition according to the present invention comprise at least one glidant chosen from a group comprising such substances as stearic acid, magnesium stearate, sodium stearyl fumarate, gliceryl behenate. Total content of glidants is preferably within a range from 0.1% to 2.5%. Optionally glidant is added in two stages, and between stages the resulting blend is sieved.

Optionally composition comprises at least one disintegrant. As disintegrant typical excipients used for this purpose may be used, including such as: sodium croscarmellose (cross-linked sodium carboxymethylcellulose), crosspovidon (cross-linked polyvinylpyrrolidone), sodium starch glycolate, starches such corn starch and modified starches. Content of disintegrant is preferably within a range from 0% to 5% by weight.

Composition according to the present invention contains optionally other additives, such as taste masking agents, pigments, flavours and other substances known by specialist, that are added to the composition in amount up to several percent without significant change of physicochemical properties of the composition.

The favourable effect being the result of the present invention is to obtain dry blend of cytisine and excipients having good technological properties. The obtained blends has good flowability and are suitable for the preparation of solid dosage forms of cytisine for oral use such as tablets, film-coated tablets, capsules and also in a form of powders for the preparation of oral suspension or powders for the preparation of oral solution in sachets.

In a particular embodiment of the present invention additional drying stage is used for the manufactured tablets, what decreases amount of impurities forming during tablet storage.

In other embodiment of the present invention the obtained tablets are put into a plastic container, where desiccator is already present, what additionally decrease amount of impurities forming during tablet storage.

Compositions prepared according to the present invention are characterized by the low level of impurities and high stability in long period of storage. Compositions prepared according to the present invention and stored in plastic containers with desiccants or in blisters made of polyvinyl chloride, polyethylene or polychlorotrifluoroethene are stable for at least one year in conditions 25°C ± 2°C/60% RH ± 5% RH.

The present invention is illustrated by the following examples of preparation:

### Example 1

Cytisine was sieved through 0.2 mm sieve and weighed. The remaining raw materials after weighing were sieved through a 0.75 mm sieve. Cytisine and 1/3 of the amount of mannitol were blended in a low-speed blender for 15 min. Then the remaining amount of mannitol was added followed by microcrystalline cellulose and blended for 15 min. Magnesium stearate was added to the obtained blend and it was blended for 3 min. The resulting blend was used for tabletting on a rotatory press, using concave punches od 7 mm diameter. The target mass of the tablet was 100 mg, and hardness above 40N. The ratios of the ingredients are presented in the table 3.

**Table 3**

| Recipe | [%] weight |
|---|---|
| cytisine | 1.5 |
| mannitol | 62.5 |
| microcrystalline cellulose | 35.0 |
| magnesium stearate | 1.0 |

Tablets having 1.51 mg/tabl. of mean content of cytisine with variance coefficient RSD 5.9% were obtained.

### Example 2

The identical procedure as in example 1 was used, but the ratios of the ingredients were changed according to the table 4. Tablets having 1.52 mg/tabl. of mean content of cytisine with variance coefficient RSD 5.6% were obtained.

**Table 4**

| Recipe | [%] weight |
|---|---|
| cytisine | 1.51 |
| mannitol | 49.49 |
| microcrystalline cellulose | 48.00 |
| magnesium stearate | 1.00 |

### Example 3

Cytisine was sieved through 0.16 mm sieve and weighed. The remaining raw materials after weighing were sieved through a 0.75 mm sieve. Cytisine, microcrystalline cellulose, sodium croscarmellose and colloidal silicon dioxide were blended in low-speed blender for 15 min. Then mannitol was added and blending was continued for 15 min. Magnesium stearate was added to the obtained blend and it was blended for 3 min. The resulting blend had good flowability and was used for tabletting on a rotatory press, using concave punches od 7 mm diameter. The target mass of the tablet was 100 mg, and hardness above 40N. The ratios of the ingredients are presented in the table 5.

**Table 5**

| Recipe | [%] weight |
|---|---|
| cytisine | 1.5 |
| mannitol | 32.0 |
| microcrystalline cellulose | 62.5 |
| colloidal silicon dioxide | 1.0 |
| croscarmellose sodium | 2.0 |
| magnesium stearate | 1.0 |

### Example 4

Micronized cytisine having average particle size d₅₀ below 20 µm was weighed. The remaining raw materials were sieved through 0.75 mm sieve. 50% of the mannitol was sieved directly to the MicroGral Duo high-shear mixer, then cytisine mixed manually with hydroxypropylmethylcellulose was added and was mixed at 50 rpm for 5 min. To the obtained blend the remaining amount of mannitol was added and blended for 5 min at 100 rpm (chopper 500 rpm), arid then the whole amount of microcrystalline cellulose was added and the mixing was continued for 10 min at 200 rpm (chopper 500 rpm). Magnesium stearate was added and blended for 3 min in low-speed cubic blender. The resulting blend was used for tabletting on a rotatory press. The ratios of the ingredients are presented in the table 6.

**Table 6**

| Recipe | [%] weight |
|---|---|
| micronized cytisine | 1.5 |
| mannitol | 72.5 |
| microcrystalline cellulose | 20.0 |
| hydroxypropylmethylcellulose | 5.0 |
| magnesium stearate | 1.0 |

Tablets having hardness of 111 N were obtained, with average mass of 100.5 mg and water content of 1.44 mg/tabl., with variance coefficient RSD 2.7%.

### Example 5

Cytisine was sieved through 0.2 mm sieve and batch amount was weighed from the fraction below 0.2 mm. Silicified microcrystalline cellulose and calcium hydrogen phosphate were weighed, followed by sieving through 0.75 mm sieve. The above mentioned ingredients (including 50% of silicified microcrystalline cellulose) were mixed portionwise in MicroGral Duo high-shear mixer at 50 rpm for 5 min. Cytisine was added to the obtained blend, followed by the remaining amount of silicified microcrystalline cellulose and the mixture was mixed for 10 min. at 200 rpm (chopper 1000 rpm). Sodium stearyl fumarate was added and blended for 5 min in the same mixer (mixing speed 200 rpm, chopper 1000 rpm). The resulting blend was used for tabletting on a rotatory press. The ratios of the ingredients are presented in the table 7.

**Table 7**

| Recipe | [%] weight |
|---|---|
| Cytisine (sieved fraction < 0.2 mm) | 1.5 |
| silicified microcrystalline cellulose | 77.5 |
| calcium hydrogen phosphate | 20.0 |
| sodium stearyl fumarate | 1.0 |

Tablets having hardness of 62 N were obtained, with average mass of 100.7 mg and cytisine content of 1.50 mg/tabl., with variance coefficient RSD 1.8%.

### Example 6

Micronized cytisine having average particle size d₅₀ of 20 µm was weighed. The remaining raw materials were weighed and sieved through 0.75 mm sieve. 70% of the mannitol was mixed with cytisine in MicroGral Duo high-shear mixer for 5 min. at 50 rpm, then the remaining amount of mannitol was added and mixed another 5 min at 50 rpm. Microcrystalline cellulose was added and mixed for 10 min at 200 rpm (chopper 500 rpm). Magnesium stearate was added and blended for 5 min in low-speed cubic blender. The resulting blend was used for tabletting on a rotatory press using concave 6 mm punches. The target mass of the tablet was 100 mg and hardness above 40 N. The ratios of the ingredients are presented in the table 8.

**Table 8**

| Recipe | [%] weight |
|---|---|
| micronized cytisine | 1.5 |
| mannitol | 62.5 |
| microcrystalline cellulose | 35.0 |
| magnesium stearate | 1.0 |

Tablets having mean cytisine content of 1.53 mg/tabl. were obtained, with variance coefficient RSD 3.4%.

### Example 7

Micronized cytisine having average particle size d₅₀ of 20 µm was weighed. The remaining raw materials were weighed and sieved through 0.75 mm sieve. 50% of the amount of mannitol was sieved to MicroGral Duo high-shear mixer, cytisine was added and mixed for 5 min. at 50 rpm. The remaining amount of mannitol and microcrystalline cellulose was added and mixed for 5 min. at 100 rpm (chopper 500 rpm). Magnesium stearate was added to the obtained blend and blended for 5 min in low-speed cubic blender. The ratios of the ingredients are presented in table 9. The blend had good technological properties. Hausner Ratio was 1.31 and Carr index 23.4%.

**Table 9**

| Recipe | [%] weight |
|---|---|
| cytisine | 1.5 |
| mannitol | 77.5 |
| microcrystalline cellulose | 20.0 |
| magnesium stearate | 1.0 |

Tablets having mean cytisine content of 1.46 mg/tabl. were obtained, with variance coefficient RSD 3.8%.

### Example 8

Micronized cytisine having average particle size d₅₀ of 20 µm was weighed. The remaining raw materials were weighed. Microcrystalline cellulose, cytisine and hydroxypropylmethylcellulose were added to low-speed cubic blender and were mixed for 15 min. The obtained blend was sieved through 0.75 mm sieve. Mannitol sieved through 0.75 mm sieve was added to the blend and mixed for 20 min. Glyceryl behenate sieved through 0.75 mm sieve was added to the blend and mixed for 10 min. The obtained blend was sieved through 0.75 mm sieve. Magnesium stearate was added and blended for 5 min. The blend had good technological properties. The ratios of the ingredients are presented in table 10. The blend had good technological properties. Hausner Ratio was 1.31 and Carr index 23.4%.

**Table 10**

| Recipe | [%] weight |
|---|---|
| cytisine | 1.5 |
| mannitol | 75.5 |
| microcrystalline cellulose | 20.0 |
| glyceryl behenate | 2.5 |
| magnesium stearate | 0.5 |

Tablets having mean cytisine content of 1.47 mg/tabl. were obtained, with variance coefficient RSD 1.7%. Loss on drying of the tablets in 105°C was 1.20%

The resulting tablets were dehumidified in the O'Hara apparatus. Loss on drying was tested with halogen moisture analyser. The following parameters were using for dehumidification process. Drum rotation: jog mode 3 rpm, drying temperature: 50°C, air flow rate: 250 m³/h, drying time 2 h.

After drying process, tablets were tested for loss on drying at 105°C, with a result of 0.75%.

### Example 9 (for better understanding the invention)

Composition prepared according to example 7 is manually encapsulated in gelatine hard capsules Cytisine content in a single capsules is in a range from 1.40 - 1.60 mg.

### Example 10

Tablets prepared according to example 7 were grouped into several parts and put into climatic stability testing chambers in conditions: 40°C/75% RH and 50°C/75% RH. The first part was packed into blisters. The second part was packed analogical as the first part, but prior to packing tablets were stored in double polyethylene bags with desiccator for 14 days.

The third part was packed into HDPE with desiccator. Tablets in bulk prepared according to example 7 were also put into chambers.

Stability testing results of cytisine content and impurities content in the tablets after 28 days storage were presented in the table 11 and 12.

**Table 11**

| **Time** | **Start** | **Day 28** | | | |
|---|---|---|---|---|---|
| **Packaging** | **in bulk** | **blisters without desiccator** | | **blisters with desiccator** | |
| **Conditions** | - | **40°C 75% RH** | **50°C 75% RH** | **40°C 75% RH** | **50°C 75% RH** |
| cytisine content | 97.2% | 96.6% | 92.0 | 95.6 | 943% |
| Sum of impurities | <0.05% | 0.17% | 1.64% | <0.05% | 0.16% |

**Table 12**

| **Time** | **Start** | **Day 28** | |
|---|---|---|---|
| **Packaging** | **in bulk** | **HDPE container with desiccator** | |
| **Conditions** | **-** | **40°C / 75% RH** | **50°C / 75% RH** |
| cytisine content | 97.2% | 97.1% | 98.7% |
| Sum of impurities | <0.05% | <0.05% | 0.05% |

### Comparative example with the use of the blend, which does not comprise microcrystalline cellulose

Micronized cytisine having average particle size d₅₀ of 20 µm was weighed. The rest of the raw materials were weighed, and then sieved through 0.75 mm sieve. Cytisine was added to the Microgral Duo high-shear mixer, followed by starch and mannitol and it was mixed for 10 min. at 100 rpm. Magnesium stearate was added and mixed for 3 min. The ratios of the ingredients used are presented in the following table:

| Recipe | [%] weight |
|---|---|
| cytisine | 1.5 |
| mannitol | 62.5 |
| starch | 35.0 |
| magnesium stearate | 1.0 |

The blend having good technological properties was obtained. The blend has a satisfactory flowability. The blend was used for tabletting, yielding tablets having average cytisine content of 1.41 mg/tabl, with variance coefficient RSD of 1.2%. Hardness of the tablets was 29 N, which was too low.

## Claims

1. Solid pharmaceutical composition comprising cytisine as an active substance, **characterized in that** said composition does not contain lactose and comprises from 20% to 75% by weight of microcrystalline cellulose, glidant and at least one pharmaceutically acceptable excipient chosen from the group comprising: mannitol, colloidal silicon dioxide, calcium hydrogen phosphate, whereby at least 60% of cytisine particles have size from 10 µm to 200 µm, and the pharmaceutical composition is in a form of tablet manufactured by direct compression method.

2. Composition according to clam 1, comprising cytisine having average particle size d₅₀ in the range from 10 µm) to 30 µm.

3. Composition according to claim 2, comprising cytisine having particle size d₉₀ below 80 µm.

4. Composition according to claim 1, **characterized in that** water content in said composition is below 1%,

5. Composition according to any of the claims 1 to 4, comprising:
a) from 0.1% to 3% by weight of the active substance;
b) from 20% to 75% by weight of microcrystalline cellulose;
c) from 20% to 77.5% by weight of diluent;
d) from 0% to 10% by weight of binder;
e) from 0% to 5% by weight of disintegrant;
f) from 0% to 2% by weight of anti-adhesion agent;
g) from 0.1% to 2.5% of glidant.

6. Composition according to any of the claims from 1 to 5, **characterized in that** anti-adhesion agent is chosen from a group comprising: colloidal silicon dioxide, hydrophilic fumed silica, talc.

7. Composition according to any of the claims from 1 to 5, **characterized in that** glidant is chosen from a group comprising: stearic acid, magnesium stearate, glyceryl behenate, sodium stearyl fumarate.

8. Composition according to any of the claims from 1 to 5, **characterized in that** binder is hydroxypropylmethylcellulose.

9. Composition according to any of the claims from 1 to 8, containing 1.5 mg of cytisine in a single tablet.

10. A process of manufacturing the solid pharmaceutical composition containing cytisine as an active substance according to claim 1, **characterized in that**:
a) cytisine, which at least 60% of particles have size from 10 µm to 200 µm is initially being blended with microcrystalline cellulose representing from 20% to 75% of the total weight of the final blend and optionally with the diluents, binders, disintegrants, anti-adhesion agents;
b) the resulting blend is being sieved through a sieve;
c) diluent chosen from a group comprising: mannitol, calcium hydrogen phosphate, colloidal silicon dioxide is added;
d) glidant chosen from a group comprising: stearic acid, magnesium stearate, glyceryl behenate, sodium stearyl fumarate is added and blended from 1 to 15 minutes;
e) optionally the resulting blend is being sieved, the second part of the glidant chosen from a group comprising: stearic acid, magnesium stearate, glyceryl behenate, sodium stearyl fumarate is added and blended from 1 to 15 minutes;
f) the obtained blend is being tabletted by the direct compression process.

11. The process according to claim 10, in which the obtained tablets are further dehumidified until reaching water content below 1%.

12. The process according to claim 10 or 11, in which the obtained tablets are packed in plastic container, that provides the protection from moisture.

13. Composition according to claim 1, **characterized in that** said composition is in a form of preparation for oral use.

## Patentansprüche

1. Feste pharmazeutische Zusammensetzung, die Cytisin als Wirkstoff enthält, **dadurch gekennzeichnet, dass** die Zusammensetzung keine Lactose umfasst und 20 bis 75 Gew.-% mikrokristalline Cellulose, Gleitmittel und mindestens einen pharmazeutisch verträglichen Hilfsstoff, ausgewählt aus der Gruppe umfassend: Mannitol, kolloidales Siliciumdioxid, Calciumhydrogenphosphat umfasst, wobei mindestens 60% der Cytisinpartikel eine Größe von 10 µm bis 200 µm aufweist, und die pharmazeutische Zusammensetzung in Form einer Tablette vorliegt, die durch ein direktes Kompressionsverfahren hergestellt wird.

2. Zusammensetzung nach Anspruch 1, umfassend Cytisin mit einer mittleren Teilchengröße d₅₀ im Bereich von 10 µm bis 30 µm.

3. Zusammensetzung nach Anspruch 2, umfassend Cytisin mit einer Teilchengröße d₉₀ unter 80 µm.

4. Zusammensetzung nach Anspruch 1, **dadurch gekennzeichnet, dass** der Wassergehalt in der genannten Zusammensetzung unter 1% liegt.

5. Zusammensetzung nach einem der Ansprüche 1 bis 4, umfassend:
a) 0,1 bis 3 Gew.-% des Wirkstoffs;
b) 20 bis 75 Gew.-% einer mikrokristallinen Cellulose;
c) 20 bis 77,5 Gew.-% eines Verdünnungsmittels;
d) 0 bis 10 Gew.-% eines Bindemittels;
e) 0 bis 5 Gew.-% eines Sprengmittels;
f) 0 bis 2 Gew.-% eines Antihaftmittels;
g) 0,1% bis 2,5% eines Gleitmittels.

6. Zusammensetzung nach einem der Ansprüche 1 bis 5, **dadurch gekennzeichnet, dass** das Antihaftmittel aus einer Gruppe umfassend: kolloidales Siliciumdioxid, hydrophile pyrogene Kieselsäure, Talkum ausgewählt ist.

7. Zusammensetzung nach einem der Ansprüche 1 bis 5, **dadurch gekennzeichnet, dass** das Gleitmittel aus einer Gruppe umfassend: Stearinsäure, Magnesiumstearat, Glycerylbehenat, Natriumstearylfumarat ausgewählt ist.

8. Zusammensetzung nach einem der Ansprüche 1 bis 5, **dadurch gekennzeichnet, dass** das Bindemittel Hydroxypropylmethylcellulose ist.

9. Zusammensetzung nach einem der Ansprüche 1 bis 8, enthaltend 1,5 mg Cytisin in einer einzelnen Tablette.

10. Verfahren zur Herstellung der festen pharmazeutischen Zusammensetzung, enthaltend Cytisin als einen Wirkstoff nach Anspruch 1, **dadurch gekennzeichnet, dass**:
a) Cytisin, von dem mindestens 60% der Teilchen eine Größe von 10 µm bis 200 µm aufweist, zunächst mit mikrokristalliner Cellulose, die 20% bis 75% des Gesamtgewichts der Endmischung darstellt, und gegebenenfalls mit der Verdünnungsmittel, Bindemittel, Sprengmittel, Antihaftmittel gemischt wird;
b) die resultierende Mischung durch ein Sieb gesiebt wird;
c) Verdünnungsmittel, ausgewählt aus einer Gruppe umfassend: Mannitol, Calciumhydrogenphosphat, kolloidales Siliciumdioxid zugegeben wird;
d) Gleitmittel, ausgewählt aus einer Gruppe umfassend: Stearinsäure, Magnesiumstearat, Glycerylbehenat, Natriumstearylfumarat zugegeben und 1 bis 15 Minuten gemischt wird;
e) gegebenenfalls die resultierende Mischung gesiebt wird, der zweite Teil des Gleitmittels ausgewählt aus einer Gruppe umfassend: Stearinsäure, Magnesiumstearat, Glycerylbehenat, Natriumstearylfumarat zugegeben und 1 bis 15 Minuten gemischt wird;
f) die erhaltene Mischung durch den direkten Kompressionsvorgang tablettiert wird.

11. Verfahren nach Anspruch 10, wobei die erhaltenen Tabletten weiter entfeuchtet werden, bis ein Wassergehalt unter 1% erreicht wird.

12. Verfahren nach Anspruch 10 oder 11, wobei die erhaltenen Tabletten in einen Kunststoffbehälter, der den Schutz vor Feuchtigkeit versieht, verpackt werden.

13. Zusammensetzung nach Anspruch 1, **dadurch gekennzeichnet, dass** die genannte Zusammensetzung in Form einer Zubereitung zur oralen Verwendung vorliegt.

## Revendications

1. Composition pharmaceutique solide comprenant de la cytisine en tant que substance active, **caractérisée en ce que** ladite composition ne contient pas de lactose et comprend de 20% à 75% en poids de cellulose microcristalline, d'agent de glissement et d'au moins un excipient pharmaceutiquement acceptable choisi dans le groupe comprenant: mannitol, dioxyde de silicium colloïdal, hydrogénophosphate de calcium, où au moins 60% des particules de cytisine ont une taille de 10 µm à 200 µm, et la composition pharmaceutique est sous forme de comprimé fabriqué par un procédé de compression directe.

2. Composition selon la revendication 1, comprenant de la cytisine ayant une taille de particule moyenne d₅₀ dans la plage de 10 µm à 30 µm.

3. Composition selon la revendication 2, comprenant de la cytisine ayant une taille de particule d₉₀ inférieure à 80 µm.

4. Composition selon la revendication 1, **caractérisée en ce que** la teneur en eau dans ladite composition est inférieure à 1%.

5. Composition selon l'une quelconque des revendications 1 à 4, comprenant:
a) de 0,1% à 3% en poids de la substance active;
b) de 20% à 75% en poids de cellulose microcristalline ;
c) de 20% à 77,5% en poids de diluant ;
d) de 0% à 10% en poids de liant ;
e) de 0% à 5% en poids de désintégrant ;
f) de 0% à 2% en poids d'agent anti-adhérence;
g) de 0,1% à 2,5% d'agent de glissement.

6. Composition selon l'une quelconque des revendications 1 à 5, **caractérisée en ce que** l'agent anti-adhérence est choisi dans un groupe comprenant dioxyde de silicium colloïdal, silice pyrogénée hydrophile, talc.

7. Composition selon l'une quelconque des revendications 1 à 5, **caractérisée en ce que** l'agent de glissement est choisi dans un groupe comprenant : acide stéarique, stéarate de magnésium, béhénate de glycéryle, stéarylfumarate de sodium.

8. Composition selon l'une quelconque des revendications 1 à 5, **caractérisée en ce que** le liant est hydroxypropylméthylcellulose.

9. Composition selon l'une quelconque des revendications 1 à 8, contenant 1,5 mg de cytisine dans un seul comprimé.

10. Un procédé de fabrication de la composition pharmaceutique solide contenant de la cytisine en tant que substance active selon la revendication 1, **caractérisé en ce que** :
a) cytisine, dont au moins 60% des particules ont une taille de 10 µm à 200 µm, est initialement mélangée avec cellulose microcristalline représentant de 20% à 75% du poids total du mélange final et éventuellement avec diluants, liants, désintégrants, agents anti-adhérence ;
b) le mélange résultant est tamisé à travers un tamis ;
c) diluant choisi dans un groupe comprenant : mannitol, hydrogénophosphate de calcium, dioxyde de silicium colloïdal est ajouté ;
d) agent de glissement choisi dans un groupe comprenant : acide stéarique, stéarate de magnésium, béhénate de glycéryle, stéarylfumarate de sodium est ajouté et mélangé de 1 à 15 minutes ;
e) éventuellement le mélange résultant est tamisé, la deuxième partie de l'agent de glissement choisi dans un groupe comprenant : acide stéarique, stéarate de magnésium, béhénate de glycéryle, stéarylfumarate de sodium est ajoutée et mélangée de 1 à 15 minutes;
f) le mélange obtenu est comprimé par le procédé de compression directe.

11. Le procédé selon la revendication 10, dans lequel les comprimés obtenus sont en outre déshumidifiés jusqu'à atteindre une teneur en eau inférieure à 1%.

12. Le procédé selon la revendication 10 ou 11, dans lequel les comprimés obtenus sont emballés dans un récipient en plastique, qui assure la protection contre l'humidité.

13. Composition selon la revendication 1, **caractérisée en ce que** ladite composition est sous la forme d'une préparation à usage oral.
